# EUROPÄISCHE PATENTSCHRIFT

(11) **EP 1 623 078 B1**
(45) Veröffentlichungstag und Bekanntmachung des Hinweises auf die Patenterteilung: **22.12.2010**
(21) Anmeldenummer: 04716513.9
(22) Anmeldetag: 03.03.2004
(51) Int. Cl.: E04B 2/14

(54) **VERFAHREN ZUM VERBINDEN VON BAUSTEINEN ZUR BILDUNG EINES BAUSTEINVERBUNDES SOWIE BAUSTEINVERBUND**
METHOD FOR BONDING BRICKS TO FORM A BRICK COMPOSITE AND CORRESPONDING BRICK COMPOSITE
PROCÉDÉ POUR ASSEMBLER DES PARPAINGS ET R ALISER UNE CONSTRUCTION DE PARPAINGS, CONSTRUCTION DE PARPAINGS

(30) Priorität: 07.03.2003 AT 3562003; 11.06.2003 AT 9022003
(43) Veröffentlichungstag der Anmeldung: 08.02.2006
(60) Teilanmeldung: 09169797.9
(73) Patentinhaber: Roitmair, Helmut, 4632 Pichl bei Wels (AT)
(72) Erfinder: Roitmair, Helmut, 4632 Pichl bei Wels (AT)
(74) Vertreter: Sonn & Partner Patentanwälte
(86) Internationale Anmeldenummer: PCT/AT2004/000065
(87) Internationale Veröffentlichungsnummer: WO 2004/079116

(56) Entgegenhaltungen:
- EP-A- 0 625 617
- WO-A-98/10030
- DE-A- 19 600 077
- DE-A- 19 801 644
- US-A- 4 312 164

## Beschreibung

Die Erfindung betrifft ein Verfahren zum Verbinden von Bausteinen mit Hohlkammern zur Bildung eines Bausteinverbundes, wobei ein im Endzustand festes Verbindungsmaterial in fließfähigem Zustand auf zumindest eine Verbindungsfläche eines Bausteins aufgebracht wird und ein zu verbindender Baustein an diese Verbindungsfläche angelegt oder aufgesetzt wird, wobei als Verbindungsmaterial ein expandierbares Kunststoffmaterial auf Basis von Polyurethan auf zumindest eine Verbindungsfläche aufgebracht wird.

Die Erfindung betrifft weiters einen Bausteinverbund mit einer Vielzahl von einzelnen, mittels eines Verbindungsmaterials in Form eines expandierten Kunststoffmaterials auf Basis von Polyurethan-Schaum miteinander verbundenen Bausteinen mit Hohlkammern.

Unter den Begriff Bausteine fallen verschiedenartigste Baumaterialien, insbesondere Ziegel, Betonsteine, Poren-Betonsteine wie z.B. Ytong-Steine, Leka-Steine, Kalksandsteine oder vieles mehr, wie sie zur Errichtung von Bauwerken oder dgl. eingesetzt werden.

Unter den Begriff Bausteinverbund fallen beispielsweise Wände oder Wandelemente, Decken oder Deckenelemente, Träger oder Stürze, Treppen oder Treppenelemente und vieles mehr. Die vorliegende Erfindung kann sowohl an der Baustelle selber als auch in einem Fertigteilwerk, wo derartige Bausteinverbunde vorgefertigt werden, Anwendung finden.

Bislang werden Bausteine, wie z.B. Ziegel, meistens mittels (silikatisch) gebundenem Material, wie Mörtel, Zement oder dergleichen, miteinander verbunden. Hydratisierungsprozesse der Ausgangsmaterialien führen zu hohen Festigkeiten und bilden eine hinreichende Haftwirkung zwischen den zu verbindenden Bausteinen. Herkömmlicherweise werden derartige Verbindungsmaterialien an der Baustelle zusammengemengt, homogenisiert und in fließfähigem Zustand auf die Verbindungsflächen der Bausteine mittels einer Kelle oder Auftragswalze flächig aufgetragen. Abgesehen von dem relativ hohen Verarbeitungsaufwand ist es aufgrund des zur Aushärtung des Materials notwendigen Wassers nicht möglich, in der kalten Jahreszeit zu arbeiten, da Mörtel beispielsweise nur bis ca. +5°C verarbeitbar ist. Demgemäß müssen Arbeiten, bei welchen ein derartiges Material verwendet wird, im Winter eingestellt werden, wodurch lange Wartezeiten in Bezug auf die Fertigstellung des Bauwerkes und damit verbundene Kosten verursacht werden. In jüngster Zeit werden zur Verbindung insbesondere von Planziegeln auch flüssige, pastöse oder Gelartige Kleber auf Zementbasis anstelle von Mörtel eingesetzt.

Seit langem gibt es im Baubereich das Bestreben, auch die kalte Jahreszeit für Arbeiten zu nutzen, um so rasch wie möglich eine Fertigstellung des Bauwerkes herbeizuführen und dadurch Zeit und Kosten einzusparen.

Zum Ausschäumen von Hohlräumen ist es in der Baubranche üblich und bekannt, Polyurethanschaum einzusetzen. Beispielsweise beschreibt die WO 94/25274 A1 ein Verfahren zum Anbringen von Dachziegeln auf eine Dachoberfläche unter Verwendung eines Zweikomponenten-Polyurethanschaums. Dabei wird der Polyurethanschaum streifenweise und relativ voluminös auf die Dachoberfläche aufgebracht und die halbkreisförmig ausgebildeten Dachziegel werden mit ihren Kanten in den Schaumstreifen eingedrückt. Durch den Schaum wird der keilförmige Spalt zwischen Dachziegel und Dachoberfläche, welcher durch das Übereinanderlegen der Dachziegel bedingt ist, ausgefüllt. Dabei resultiert ein relativ hoher Materialaufwand und eine nur begrenzte Haftfähigkeit, welche bei Bausteinverbunden, wie z.B. Wänden, zu niedrig wäre.

Die US 3 839 519 A beschreibt ein Verfahren zur Herstellung von Wänden für ein Fertigteilhaus, wobei mehrere Ziegel mittels Polyestermörtel aneinandergefügt werden. Zur Erzielung der notwendigen Festigkeit der Fertigteile wird an der Rückseite der Wand ein Stahlnetz angebracht, an welches die Innenwand positioniert wird. Zwischen der Innenwand und dem mit Polyestermörtel ausgefüllten Stahlnetz wird Polymerschaum eingebracht. Der Schaum dient dabei als Isoliermaterial, die Festigkeit wird durch das Stahlnetz erzielt. Abgesehen vom relativ hohen Herstellungsaufwand ist dieses Verfahren nur in Fertigteilwerken und nicht direkt an der Baustelle anwendbar.

Der Aufwand für die Vorfertigung solcher Bausteinverbunde, welche eine sehr hohe Tragfähigkeit aufweisen müssen, wie z.B. Stürze für die Erstellung der oberen Begrenzung von Durchbrüchen, Fenster- und Türöffnungen oder dgl. in Wänden, Deckenelemente zur Herstellung von Balkonen oder dgl. Stiegenaufgänge, ist derzeit relativ groß. Fenster- oder Türstürze und Deckenelemente werden heutzutage meist aus Beton mit Stahlarmierungen hergestellt und mit einer Ziegelumhüllung versehen, um eine Homogenität in einer Ziegelwand zu erzielen. Abgesehen vom erwähnten Herstellungsaufwand weisen derartige Bausteinverbunde ein relativ hohes Gewicht auf, wodurch der Transport zur Baustelle und die folgende Handhabung erschwert wird. Darüber hinaus bewirkt die Betonfüllung eine schlechte Wärmedämmung.

Um das Gewicht derartiger Bausteinverbunde in Grenzen zu halten, weisen diese üblicherweise eine geringere Höhe als die Bausteine zur Herstellung des Mauerwerkes eines Gebäudes auf. Dies erschwert wiederum die Herstellung von Wänden mit Tür- oder Fensteröffnungen oder dgl., da beispielsweise die Ziegelreihe über einem Sturz durch entsprechende Ziegel mit geringerer Höhe hergestellt werden muss, um wieder auf die normale Ziegelhöhe zu gelangen. Somit resultieren erhöhte Bauzeiten und in der Folge höhere Baukosten. Ein Beispiel für derartige vorgefertigte Deckenelemente oder Stürze für Fenster- oder Türöffnungen gibt die EP 970 883 A2.

Die AT 397 528 B zeigt einen Bausteinverbund in Form einer Ziegelüberlage, deren Höhe jener von Hohllochziegeln entspricht und die leicht hantierbar und stapelbar ist und darüber hinaus auch gute Wärmedämmeigenschaften aufweist. Zu diesem Zweck weist die Ziegelüberlage einen besonderen Querschnitt mit mehreren Hohlräumen auf, in welchen Vorspannelemente aus Eisen angeordnet sind, und welche vorzugsweise mit Beton ausgegossen sind. Darüber hinaus existiert ein Wärmedämmbereich mit zumindest einer Hohlkammer. Eine derartige Ziegelüberlage ist allerdings weiterhin relativ aufwendig in der Herstellung und weist durch die Betonfüllung relativ hohes Gewicht auf.

Ein anderer Bausteinverbund in Form eines Sturzelements für ein Ziegelmauerwerk ist aus der DE 196 28 963 A1 bekannt, wobei Ziegelsteine mit dazwischen angeordneten Abstandhaltern über Zugstangen miteinander verspannt werden. Als Abschluss werden winkelartig ausgebildete Auflageelemente angeordnet, über die das Sturzelement auf die Seitenwände einer Fensteröffnung oder dgl. aufgelegt wird. Dieses Sturzelement ist zwar wegen der fehlenden Betonausgießung leichter als herkömmliche Sturzelemente, weist jedoch aufgrund der Zugstangen noch immer relativ hohes Gewicht auf und ist relativ aufwendig in der Herstellung.

Die WO 98/10030 A1 welche den Oberbegriff des zweiteiligen Anspruchs offenbart betrifft einen 2-Komponenten-Klebstoff auf Basis von Polyurethan, der eine besonders schnelle Aushärtezeit und hohe Klebkraft aufweist. Zu diesem Zweck umfasst der 2-Komponenten-Klebstoff eine Komponente, die wenigstens ein Polyol enthält und eine weitere Komponente, welche wenigstens ein Isocyanat enthält. Dieses Dokument offenbart zwar das grundsätzliche Verfahren zum Verbinden von Bausteinen zur Bildung eines Bausteinverbundes bzw. einen Bausteinverbund mit einer Vielzahl von einzelnen mittels eines auf Polyurethan-Schaum basierenden Verbindungsmaterials miteinander verbundenen Bausteinen, ohne dass jedoch auf die Beschaffenheit der Verbindungsflächen der Bausteine näher eingegangen wird. Gerade diese Beschaffenheit der Verbindungsflächen der Bausteine ist jedoch für die Festigkeit des resultierenden Bausteinverbundes wesentlich.

Die DE 196 00 077 A1 betrifft einen Bausteinverbund zum Erstellen von Wänden oder Mauern, bei denen Dünnbettmörtel als Verbindungsmaterial verwendet wird. Um zu verhindern, dass die vertikalen Hohlkammern der Bausteine offen bleiben, wodurch es zu Schallübertragungen im Mauerwerk kommen kann, ist vorgesehen, dass zwischen den einzelnen Lagen der Bausteine ein Verbindungsband angeordnet wird, welches beidseits mit dem Verbindungsmaterial beschichtet ist. Das Verbindungsband kann aus Leinwand, einer Sisalmatte oder einer Kunststofffolie gebildet sein. Mit einem derartigen Verbindungsband können keine hohen Festigkeiten des entstehenden Bausteinverbundes erzielt werden. Darüber hinaus ist durch die Verwendung von Dünnbettmörtel die Anwendung bei tiefen Temperaturen ausgeschlossen.

Die US 4 312 164 A beschreibt einen isolierenden Bausteinverbund, bei dem für eine optimale Isolierung Isolierschichten speziell angeordnet sind.

Die DE 198 01 644 A1 beschreibt einen Baustein mit einem sich zwischen der Grund und der Deckfläche erstreckenden Hohlraum, der bezüglich der Mittelebene zwischen der Innenfläche und der Außenfläche des Bausteins außermittig versetzt zur Außenfläche benachbart angeordnet ist.

Schließlich zeigt die EP 625 617 A1 ein Verfahren zur Herstellung eines Bausteinverbunds, bei welchem zwischen den einzelnen Bausteinen Isolierschichten angeordnet werden, um optimale Wärmeisolation zu erzielen.

Zusammenfassend ergibt sich daher, dass bekannte Verfahren zum Verbinden von Bausteinen zur Bildung eines Bausteinverbundes relativ zeit- und kostenintensiv sind und bzw. oder die dadurch hergestellten Bausteinverbunde eine unzureichende Festigkeit aufweisen.

Die Aufgabe der vorliegenden Erfindung besteht daher in der Schaffung eines Verfahrens zum Verbinden von Bausteinen, welches möglichst einfach, rasch und kostengünstig durchführbar ist und vorzugsweise auch bei tieferen Temperaturen angewendet werden kann, so dass ein Bauwerk auch während der kalten Jahreszeit aufgebaut werden kann. Die Festigkeit derartig hergestellter Bausteinverbunde soll möglichst hoch sein, um auch den Aufbau tragender Bausteinverbunde zu ermöglichen. Schließlich soll zum Sparen der Kosten für das Verbindungsmaterial dessen Verbrauch bei der Verbindung der Bausteine möglichst gering sein.

Eine weitere Aufgabe der vorliegenden Erfindung besteht in der Schaffung eines Bausteinverbunds, der möglichst stabil ist, möglichst geringes Gewicht aufweist, und die bautechnischen und baurechtlichen Auflagen für Bauwerke, insbesondere für Gebäude, erfüllt. Darüber hinaus soll der Bausteinverbund in Bezug auf Schall- und Wärmedämmung gute Eigenschaften aufweisen. Schließlich soll der Bausteinverbund möglichst einfach, rasch und kostengünstig herstellbar sein und möglichst einfach und rasch im Bauwerk integrierbar sein. Nachteile bekannter Konstruktionen sollen vermieden oder zumindest reduziert werden.

Die erste Aufgabe der Erfindung wird in verfahrensmäßiger Hinsicht dadurch gelöst, dass die zu verbindenden Bausteine mit derartigen Verbindungsflächen ausgestattet und aneinander gereiht werden, dass sich ein Klebespalt mit im Wesentlichen konstanter Breite von kleiner 1 mm ergibt, und dass gleichzeitig mit dem Aufbringen des Verbindungsmaterials auf zumindest eine Verbindungsfläche des zu verbindenden Bausteins auch die Hohlräume in den Bausteinen mit dem Verbindungsmaterial befüllt werden. Gemäß der Erfindung wird unter expandierbarem Kunststoffmaterial ein vor bzw. während der Verarbeitung expandierbares bzw. aufschäumendes Material verstanden, das anschließend aushärtet. Vor der Aushärtung ist das Kunststoffmaterial klebefähig. Beispielsweise weist das Material ein Prepolymer auf, das durch Polyaddition oder Polymerisation expandiert. Günstigerweise findet die Reaktion unter Einfluss von Luftsauerstoff bzw. Luftfeuchtigkeit statt, so dass kein Additiv zugefügt werden muss. Die Vorteile von expandierfähigem Kunststoffmaterial zur Verbindung von Bausteinen bestehen sowohl in einer einfachen Verarbeitbarkeit als auch in den hervorragenden Materialeigenschaften im ausgehärteten Zustand. Nicht nur die Einsatzmöglichkeit bei tieferen Temperaturen ist für den Baustellenbetrieb zur kalten Jahreszeit von Vorteil, sondern auch die Tatsache, dass keine aufwändigen Vorarbeiten, wie Mischen, zeitaufwändiges Homogenisieren, etc., durchgeführt werden müssen, um das Material einsatzfähig zu machen. Beispielsweise kann das Kunststoffmaterial in unter Druck stehenden Behältern an die Baustelle geliefert und von dort ohne Aufwand verarbeitet werden. Dies hat zum einen den Vorteil, dass durch solche Behälter die Expansion des Kunststoffmaterials gefördert werden kann und zum anderen, dass das Kunststoffmaterial sogleich in dosierten Mengen auf die Oberfläche der zu verbindenden Bausteine aufgebracht werden kann. Zum Beispiel kann es direkt auf die Verbindungsfläche aufgespritzt werden, was bei relativ kurzen Reaktionszeiten des Kunststoffmaterials wichtig ist. Dadurch wird der Materialverbrauch auf ein Minimum reduziert. Im Falle einer zur Verfügungstellung in unter Druck stehenden Behältern ist es hinsichtlich der Umweltfreundlichkeit günstig, wenn es frei von FCKW, HFCKW und HFKW ist. PUR-Schaum wird derzeit zum Abdichten von Hohlräumen, wie zum Beispiel beim Einsetzen von Fenstern, verwendet. Ein derartiges Kunststoffmaterial umfasst bekannterweise Diphenylmethan-Diisocyanat, Methylen-Diphenyl-Diisocyanat oder dergleichen. Des Weiteren sind Isomere, Homologe und Ähnliches enthalten. Die Vorteile des PUR-Schaums bestehen nicht nur in seiner einfachen Verarbeitbarkeit, sondern auch in seinen Eigenschaften, wie eine ausgezeichnete Haftung auf diversen Materialflächen. Dies wird erfindungsgemäß für eine starke und dauerhafte Verbindung von Bausteinen ausgenutzt. Ferner erfüllt ein PUR-Schaum die normtechnischen Anforderungen hinsichtlich des Dämmverhaltens. Dies hat nicht nur zur Folge, dass eine wesentlich bessere Dämmung erreicht werden kann, sondern auch, dass zusätzliche Mittel zur Dämmung von Bauwerken eingespart werden können. Bei Aufbringung des expandierbaren Kunststoffmaterials auf eine Verbindungsfläche eines Bausteins kann dieser in die Poren des Bausteins eindringen, wodurch die Schall- sowie auch die Wärmeleitung über die Poren im Baustein verringert bzw. sogar unterbunden werden. Insbesondere in Bezug auf die Wärmebrücken, welche große Schwachstellen an Außenwandbauteilen darstellen, ist ein Verbindungsmaterial, wie PUR-Schaum von Vorteil. Der Vorteil liegt nicht nur in der Energieeinsparung, sondern auch in der Vermeidung einer Materialkorrosion infolge von Tauwasserbildungen an der Innenseite der betroffenen Bauteile, was im schlimmsten Fall zu Bauschäden, Schimmelbildungen, Rissen und dgl., führen kann. Dies sind nur einige wenige Vorteile, welche einen Einsatz von PUR-Schaum gegenüber herkömmlichen Klebern, wie beispielsweise Klebemörtel, insbesondere mit Zement, begünstigen. Die geforderte Festigkeit der Verbindung wird einerseits durch die Art des Verbindungsmaterials und andererseits die Beschaffenheit der Verbindungsflächen der Bausteine erzielt. Die Verbindungsflächen sind nämlich derart ausgestattet, dass die gegenüberliegenden Verbindungsflächen der aneinander gereihten Bausteine im Wesentlichen parallel zueinander angeordnet sind, so dass sich ein Klebespalt ergibt, der im Wesentlichen konstante und möglichst geringe Breite von vorzugsweise kleiner 1 mm. Neben der besseren Festigkeit ist auch der Verbrauch des Verbindungsmaterials bei geringer Klebespaltbreite minimal. Die Dämmung der miteinander verbundenen Bausteine wird dadurch optimiert, dass mit dem Verbindungsmaterial auch Hohlräume in den Bausteinen befüllt werden. Das expandierte ausgehärtete Kunststoffmaterial hat nämlich auch gute Dämmeigenschaften. Insbesondere wenn die Befüllung der Hohlräume mit dem Verbindungsmaterial gleichzeitig mit dem Aufbringen des Verbindungsmaterials auf zumindest eine Verbindungsfläche des zu verbindenden Bausteins erfolgt, hat dies hinsichtlich Zeit-, Material- und Kostenersparnis Vorteile. Einige Arbeitsschritte (und auch dafür erforderliche Materialien) auf der Baustelle können eingespart werden, welche herkömmlicherweise darin bestehen die Hohlräume der Bausteine, beispielsweise mit Styroporkörpern, zu befüllen.

Um einen Klebespalt mit im Wesentlichen konstanter und möglichst geringer Breite zu erzielen ist es von Vorteil, wenn zumindest eine Verbindungsfläche, vorzugsweise jede Verbindungsfläche jedes zu verbindenden Bausteins vor dem Aufbringen des Verbindungsmaterials geschliffen wird. Dies ist insbesondere bei Ziegeln von Vorteil, die nach dem Brennen einem gewissen Schwund unterliegen, so dass eine nicht ebene Verbindungsfläche resultiert, was beim Verbinden der Bausteine zu Klebespalten führen würde, welche unterschiedliche Breite aufweisen. Dadurch würde die Festigkeit des Bausteinverbunds reduziert und gleichzeitig der Verbrauch des Verbindungsmaterials erhöht. Bei anderen Bausteinen, wie z.B. Bausteinen aus Porenbeton, kann eine Nachbearbeitung des Bausteins nach dessen Herstellung unterbleiben, wenn die Verbindungsflächen ohnedies besonders glatt bzw. eben ausgebildet sind. Es wird darauf hingewiesen, dass es sich bei den Verbindungsflächen nicht um ebene Flächen handeln muss, sondern auch anders gestaltete Verbindungsflächen möglich sind, welche eine Verbindung zweier Bausteine in Art einer Nut-Feder-Verbindung bilden. Wesentlich bei der Verbindung ist, dass der sich ergebende Klebespalt im Wesentlichen konstante Breite aufweist und möglichst schmal ist. Die Feinheit des Schliffs des Bausteins kann an das verwendete Material des Bausteins und an die Anforderungen des Bausteinverbunds angepasst werden.

Gemäß einem weiteren vorteilhaften Merkmal der Erfindung besteht das Verbindungsmaterial aus zumindest zwei Komponenten, welche vor dem Aufbringen auf die Verbindungsfläche in Kontakt gebracht werden. Ein solches Verbindungsmaterial hat den Vorteil, dass sowohl die chemischen als auch die technologischen Charakteristika eines Einkomponenten-Verbindungsmaterials beeinflusst werden können. Die Verarbeitbarkeit des Verbindungsmaterials kann weiters verbessert werden, wenn das expandierbare Kunststoffmaterial aus zumindest zwei Komponenten besteht, welche vor dem Aufbringen auf die Oberfläche der Bausteine in Kontakt gebracht werden. Das Zusammenführen der beiden Komponenten kann beliebig gestaltet sein, wobei hiezu beispielsweise ein unter Druck stehender Behälter mit zwei Kammern für die beiden Komponenten herangezogen werden kann. Ein Zusammenführen der zwei Komponenten kann zum Beispiel in einem Spritzkopf dieses Behälters stattfinden und das so homogenisierte Material auf die Oberfläche der zu verbindenden Bausteine in dosierten Mengen aufgebracht werden.

Zusätzlich können dem Verbindungsmaterial Zusätze, wie Stabilisatoren, Katalysatoren, alterungs-, flammhemmende Mittel oder dergleichen beigemengt werden.

Je nach Anforderung an die Festigkeit der Verbindung kann das Verbindungsmaterial punktuell, entlang zumindest einer Linie oder auch flächig aufgebracht werden. Gegenüber einer punktuellen Anordnung des Verbindungsmaterials hat die Anordnung in einer Linie oder auf einer Fläche in Bezug auf Wärme- und Lärmschutz einen Vorteil, da eine durchgehende Verbindung geschaffen wird und eine Ritzenbildung für eine Wärme- oder Schallübertragung vermieden werden kann. Hinsichtlich der Druckbelastung ist es immer günstig, das Kunststoffmaterial möglichst dünn aufzutragen. Zur Auftragung des Kunststoffmaterials können Hilfsmittel, wie beispielsweise Schablonen, herangezogen werden.

Für eine bessere Verbindung bzw. Haftung der einzelnen Bausteine ist es gemäß der Erfindung vorteilhaft, wenn die Verbindungsfläche des zu verbindenden Bausteins vor dem Aufbringen des Verbindungsmaterials vorbehandelt wird. Beispielsweise kann die Oberfläche befeuchtet werden. Die Haftung kann weiters verbessert werden, indem vor Auftragung des expandierbaren Kunststoffmaterials die Verbindungsflächen von losen Teilen, Schmutz und dgl. befreit werden, so dass sauber gearbeitet werden kann.

Um die Dämmeigenschaften des Bausteinverbunds weiter zu erhöhen, können die Bausteine mit zumindest einer Isolierschicht verbunden werden, wobei als Verbindungsmaterial vorzugsweise das expandierbare Kunststoffmaterial verwendet wird. Die Verwendung dieses Verbindungsmaterials gewährleistet einerseits den guten Halt der Isolierschicht an den Bausteinen und erleichtert auch die Arbeitsschritte, da sowohl zur Verbindung der Bausteine untereinander als auch zur Verbindung der Bausteine mit der Isolierschicht das selbe Verbindungsmaterial verwendet wird.

Um optimale Dämmeigenschaften des Bausteinverbunds zu erzielen ist vorgesehen, dass die Bausteine zu einem mehrschaligen Bausteinverbund verbunden werden, wobei zwischen den einzelnen Bausteinverbunden zumindest eine Isolierschicht angeordnet wird, wobei die Isolierschicht mit den Bausteinen und allenfalls mehrere Isolierschichten untereinander mit dem expandierbaren Kunststoffmaterial verbunden werden. Dadurch können Bausteinverbunde aufgebaut werden, welche neben einer hervorragenden Festigkeit auch hervorragende Dämmeigenschaften aufweisen. Je nach verwendetem Material für die Isolierschicht muss diese zur Erzielung eines möglichst niedrigen U-Werts eine mehr oder weniger große Dicke aufweisen.

Insbesondere bei der Verwendung des Bausteinverbunds als Träger, Sturz-, Deckenelement oder dgl. ist es von Vorteil, wenn der durch die Bausteine gebildete Bausteinverbund durch Aufbringen von Verstärkungselementen, wie z.B. von Verstärkungsgeweben oder Verstärkungsleisten entlang zumindest einer Oberfläche des Bausteinverbunds verstärkt wird. Die Verstärkungselemente wie Verstärkungsgewebe oder Verstärkungsleisten können beispielsweise aus Glasfaser-verstärktem Material bestehen und halten den Bausteinverbund zusammen.

Insbesondere bei der Verwendung von langgestreckten Verstärkungselementen, wie z.B. Verstärkungsleisten kann es von Vorteil sein, wenn in die Oberfläche des Bausteinverbunds eine Nut oder dgl. eingebracht wird, in welche das Verstärkungselement bzw. die Verstärkungsleiste eingelegt wird. Somit wird durch die Anordnung des Verstärkungselements die im Wesentlichen plane Oberfläche des Bausteinverbunds nicht beeinträchtigt.

Die Verstärkungselemente werden mit dem Bausteinverbund vorteilhafterweise verklebt, wobei ebenso das expandierbare Kunststoffmaterial verwendet werden kann.

Die zweite Aufgabe der vorliegenden Erfindung wird durch einen oben genannten Bausteinverbund gelöst, wobei der Klebespalt zwischen zwei verbundenen Bausteinen eine im Wesentlichen konstante Breite von maximal 1 mm aufweist, und dass das Verbindungsmaterial auch die Hohlkammern der Bausteine befüllt. Der Klebespalt soll für optimale Festigkeits- und Dämmeigenschaften des Bausteinverbunds möglichst gering sein. Bei sehr präzise gefertigten bzw. plangeschliffenen Bausteinen kann die Breite des Klebespalts gegen Null gehen. Das zur Verbindung der Bausteine verwendete Material dringt in die Hohlkammern der Bausteine ein, wodurch eine höhere Haftung in Folge der vergrößerten Kontaktfläche des Bausteins mit dem Verbindungsmaterial erzielt wird. Hinsichtlich der Festigkeit ist das in die Hohlkammern eingedrungene Verbindungsmaterial insoweit vorteilhaft, da die Bereiche, in denen Kohäsionskräfte wirken, erhöht werden können. Darüber hinaus ist dies im Bezug auf die Dämmeigenschaften des Bausteinverbundes günstig, da die Wärme- und Schallbrücken durch die größere Verteilung des Verbindungsmaterials am Baustein verstärkt minimiert werden. Aufgrund der ausgezeichneten Dämmeigenschaften der Materialien, insbesondere von Polyurethanschaum, ist es möglich herkömmliche Dämmmaterialen einzusparen. Zudem stellt diese Ausgestaltung auch eine Verminderung von Arbeitsschritten beim Herstellen eines Bausteinverbundes dar.

Vorteilhafterweise weisen die Bausteine des Bausteinverbunds zumindest eine geschliffene Verbindungsfläche auf. Dies ist nicht nur hinsichtlich geringerer Fugen zwischen den einzelnen Bausteinen und infolgedessen besserer Dämmung des gesamten Bausteinverbundes günstig, sondern auch hinsichtlich eines minimierten Materialerfordernisses. Zusätzlich ist eine geschliffene Verbindungsfläche in Bezug auf die Druckbelastungen vorteilhaft, welchen die einzelnen Bausteine im Bausteinverbund ausgesetzt sind.

Die Bausteine können mit einem Mehrkomponentenmaterial verbunden sein.

Zusätzlich können die Bausteine an den Verbindungsflächen Öffnungen zur Aufnahme des Verbindungsmaterials aufweisen. Durch diese Öffnungen an den einander zugewandten Verbindungsflächen der Bausteine wird erreicht, dass das expandierbare Kunststoffmaterial einen entsprechenden Halt findet und somit eine extrem stabile Verbindung zwischen den Bausteinen ermöglicht. Die Kombination des verwendeten Verbindungsmaterials und der entsprechenden Gestaltung der Bausteine an den Verbindungsflächen ermöglicht die Herstellung von Bausteinverbunden, welche eine extrem hohe Tragfähigkeit aufweisen, ohne dass zusätzliche Verstärkungselemente, wie Vorspannelemente, aus Eisen und bzw. oder Bereiche, welche mit Beton ausgegossen werden, notwendig sind. Somit resultiert einerseits ein geringes Gewicht des Bausteinverbunds, was die Handhabung und den Transport wesentlich erleichtert. Zusätzlich entsprechen die Wärmedämmeigenschaften eines derartigen Bausteinverbunds im Wesentlichen jenen der verwendeten Bausteine und werden nicht durch Wärmebrücken aufgrund von Betoneinschlüssen oder dergl. herabgesetzt. Die Herstellung derartiger Bausteinverbunde ist besonders rasch und kostengünstig möglich, da die Bausteine lediglich miteinander verklebt werden müssen. Dies kann sowohl manuell als auch automatisiert erfolgen. Zudem weisen derartige Bausteinverbunde im Brandfall besonders gute Eigenschaften auf, da es nicht wie bei herkömmlichen Bausteinverbunden, wie zum Beispiel Stürzen oder dergl. zu einem Bruch des Sturzes aufgrund unterschiedlicher Ausdehnungskoeffizienten von Ziegel, Eisenarmierung und Beton kommen kann. Die Öffnungen an den Verbindungsflächen der Bausteine können einfach durch Bohrungen oder Schlitze gebildet werden. Diese können nach der Herstellung des Bausteins vorzugsweise automatisiert hergestellt werden. Die Öffnungen können über einen Teilbereich oder auch die gesamte Höhe des Bausteins reichen.

Vorteilhafterweise reichen die Öffnungen an den Verbindungsflächen der Bausteine zumindest teilweise bis zu Hohlkammern der Bausteine und ist das Verbindungsmaterial zumindest teilweise in den Hohlkammern angeordnet. Dadurch kann sich das expandierbare Material des Verbindungsmaterials über die Öffnungen bis in die Hohlkammern ausdehnen und somit eine besonders hohe Zugfestigkeit entwickeln. Das Verbindungsmaterial baut somit über die Öffnungen und die Hohlkammern eine Art Widerlager auf.

Insbesondere bei der Verwendung eines derartigen PolyurethanSchaums ist es zweckmäßig, dass zumindest die mit den Öffnungen an den Verbindungsflächen der Bausteine verbundenen Hohlkammern vollständig mit Verbindungsmaterial ausgefüllt sind. Dadurch wird nicht nur die Zugfestigkeit des Bausteinverbunds erhöht, sondern auch das Wärme- und Schalldämmverhalten verbessert.

Um die Tragfähigkeit derartiger Bausteinverbunde noch weiter zu erhöhen, ist zumindest an einer Oberfläche der miteinander verbundenen Bausteine zumindest ein Verstärkungselement, beispielsweise ein Verstärkungsgewebe oder eine Verstärkungsleiste angeordnet und mit diesem verklebt. Das Verstärkungselement schützt den Bausteinverbund auch während des Transports vor Beschädigung aufgrund unzulässiger Krafteinwirkungen.

Das Verstärkungselement kann durch ein Glasfasergewebe gebildet sein, das einerseits leicht und gut an der Oberfläche der verbundenen Bausteine aufklebbar ist und andererseits gute Festigkeitseigenschaften aufweist und zudem schwer brennbar ist.

An der Oberfläche des Bausteinverbunds, an der das zumindest eine Verstärkungselement angeordnet ist, kann eine Nut oder dergl. zur Aufnahme des Verstärkungselements, insbesondere der Verstärkungsleiste, angeordnet sein.

Noch bessere Eigenschaften werden dann erzielt, wenn jeweils zumindest ein Verstärkungselement sowohl an der Oberseite als auch an der Unterseite der miteinander verbundenen Bausteine angeordnet ist. Dabei kann das Aufkleben der Verstärkungselemente herstellungsseitig oder auch erst an der Baustelle erfolgen. Durch Übereinanderstapeln mehrerer Bausteinverbunde mit dazwischen angeordneten Vestärkungselementen können für besondere Anforderungen Elemente mit noch höherer Tragfähigkeit hergestellt werden. Abgesehen von Glasfasergeweben können auch andere Materialien, wie z.B. Kunststoffgewebe aus Carbonfasern, Keflar oder dergl., für die Verstärkungselemente eingesetzt werden.

Zur Bildung eines Sturzes, Trägers oder dergl. werden die Bausteine in einer Richtung aneinandergereiht. Auf diese Weise können, wie bereits erwähnt, Stürze zur Bildung des oberen Abschlusses von Wandöffnungen, wie z.B. Fenster- oder Türöffnungen, aber auch Träger für Balkone oder dgl., gebildet werden.

Dabei ist es von Vorteil, wenn die Höhe des Bausteinverbunds im Wesentlichen der Höhe angrenzender Bausteine entspricht. Dadurch wird eine rasche Herstellung von Bauwerken mit entsprechenden Wandöffnungen ermöglicht, da die Baustein-Lage ober dem Sturz oder dergl. nicht mit besonders angefertigten oder zurechtgeschnittenen Bausteinen ergänzt werden muss, sondern gleich mit den Bausteinen, wie z.B. Ziegeln, mit Normhöhe weitergebaut werden kann.

Wenn die Bausteine in einer Ebene aneinander angeordnet werden, können Deckenelemente oder dergl. hergestellt werden. Flächige Bausteinverbunde können auch durch Aneinanderreihen mehrerer oben erwähnter Stürze, Träger oder dergl., allenfalls unter Zwischenlage von Verstärkungsschichten, gebildet werden.

Weiters ist es möglich, durch teilweise überlappende Anordnung entsprechender Bausteine auch Treppenelemente zu bilden.

Um Bausteinverbunde mit besseren Dämmeigenschaften zu erhalten, ist vorgesehen, dass mit den Bausteinen zumindest eine Isolierschicht mit dem expandierbaren Kunststoffmaterial verbunden ist.

Weiters kann zwischen Bausteinen zumindest eine Isolierschicht angeordnet sein, wobei die Isolierschicht mit den Bausteinen und allenfalls mehrere Isolierschichten untereinander mit dem expandierbaren Kunststoffmaterial verbunden sind. So können zweischalige bzw. mehrschalige Bausteinverbunde hergestellt werden, welche neben einer ausgezeichneten Stabilität auch ausgezeichnete U-Werte aufweisen.

Die vorliegende Erfindung wird anhand der beigefügten Abbildungen näher erläutert. Darin zeigen:
- Fig. 1: eine perspektivische Ansicht eines aus mehreren Baustei- nen aufgebauten Bausteinverbunds;
- Fig. 2: einen Schnitt durch einen Teil eines Bausteinverbunds im Bereich der Verbindung zweier Bausteine;
- Fig. 3a: und Fig. 3b Schnittbilder durch Bausteinverbunde im Be- reich der Verbindung zweier Bausteine gemäß der vorlie- genden Erfindung;
- Fig. 4: einen Schnitt durch einen zweischaligen Bausteinverbund;
- Fig. 5: einen Schnitt durch einen zweischaligen Bausteinverbund mit dazwischen angeordneter Isolierschicht;
- Fig.6: schematisch eine Anordnung eines herkömmlichen Fenster- sturzes in einer Hauswand mit einer Fensteröffnung;
- Fig.7: schematisch die Anordnung eines als Fenstersturz ausge- bildeten erfindungsgemäßen Bausteinverbunds in einer Hauswand mit einer Fensteröffnung;
- Fig.8: eine perspektivische Ansicht eines durch eine Reihe von miteinander verbundenen Bausteinen gebildeten Baustein- verbunds;
- Fig.9: einen horizontalen Schnitt durch einen Bausteinverbund gemäß Fig.8 in vergrößerter Darstellung im Bereich der Verbindung zweier Bausteine;
- Fig.10: und Fig.11 zwei Ausführungsformen der Öffnungen an den Verbindungsflächen der Bausteine;
- Fig. 12: und 13 perspektivische Ansichten weiterer Beispiele für erfindungsgemäße Bausteinverbunde;
- Fig. 14: eine perspektivische Ansicht eines Bausteinverbunds mit daran angeordneten Verstärkungsleisten;
- Fig. 15: einen Schnitt durch den Bausteinverbund gemäß Fig. 14;
- Fig. 16: eine Variante gegenüber Fig. 15;
- Fig. 17: einen flächigen Bausteinverbund; und
- Fig. 18: ein Schnittbild eines Bausteinverbunds in Form einer Treppe bzw. eines Treppenelements.

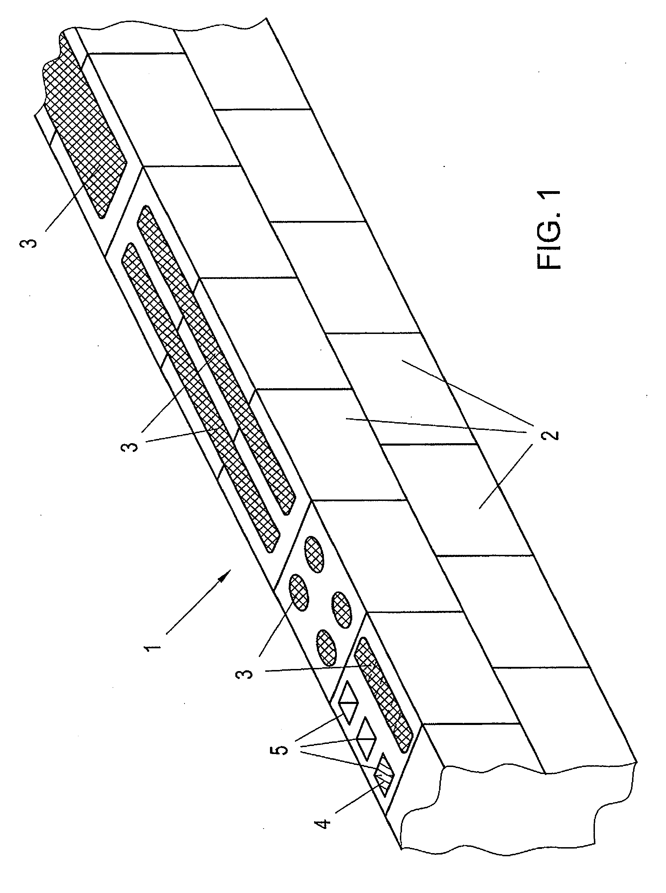

Die Fig. 1 zeigt einen Bausteinverbund 1 aus miteinander verbundenen Bausteinen 2, wie zum Beispiel Ziegeln Die Bausteine 2 sind in üblicher Weise versetzt aufeinandergelegt, wobei als Verbindungsmaterial 3 expandierbares Kunststoffmaterial verwendet wird. Das Verbindungsmaterial 3 kann flächig, entlang Linien, oder punktuell aufgetragen werden. Das Auftragen des Verbindungsmaterials 3 kann beispielsweise mit einer Pistole oder dergl., durchgeführt werden. Der Baustein 2 weist Hohlkammern 5 auf, welche mit dem Verbindungsmaterial 3 oder einem anderen Dämmmaterial 4 befüllt sein können. Die Zahl und Form der Hohlkammern 5 kann beliebig sein. Ebenso müssen nicht alle Hohlkammern 5 mit dem Verbindungsmaterial 3 oder einem anderen Dämmmaterial gefüllt sein. Erfindungsgemäß sind die Verbindungsflächen der Bausteine 2 derart ausgebildet, dass sich ein Klebespalt mit im Wesentlichen konstanter Breite und möglichst geringer Breite ergibt.

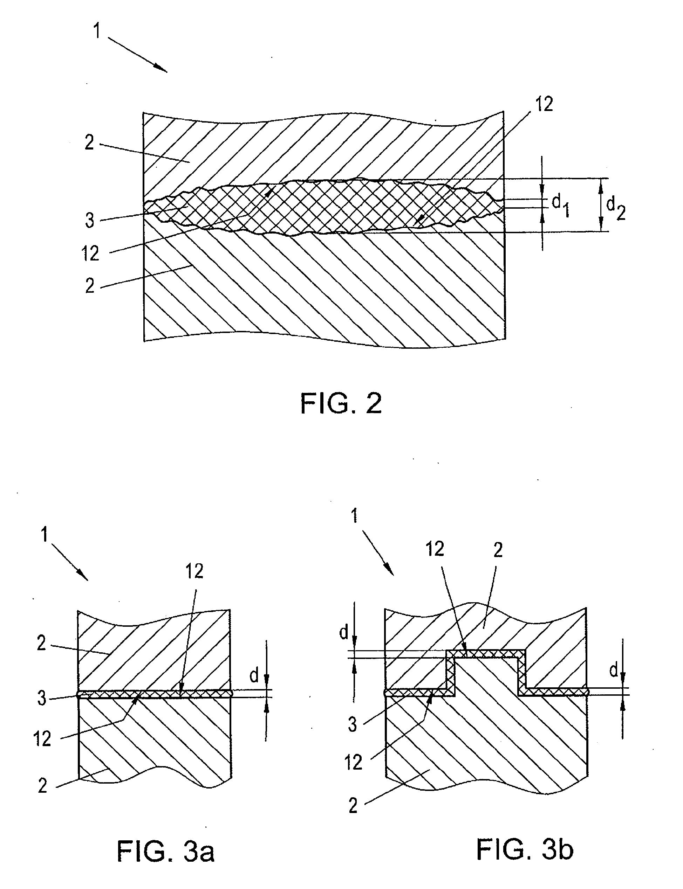

Fig. 2 zeigt ein Schnittbild durch einen Bausteinverbund 1 im Bereich der Verbindung zweier Bausteine 2, wie er gemäß dem Stand der Technik auftritt. Die Bausteine 2, insbesondere Ziegel, weisen meist relativ unregelmäßige Verbindungsflächen 12 auf, resultieren in einem Klebespalt mit unregelmäßiger Breite d, welche von Minimalwerten d₁ bis zu Maximalwerten d₂ reichen. Dadurch, dass die Verbindungsflächen 12 der zu verbindenden Bausteine 2 nicht parallel angeordnet sind, wird einerseits der Materialverbrauch für das Verbindungsmaterial 3 erhöht, andererseits die Festigkeit des Bausteinverbunds 1 verringert. Insbesondere bei der Herstellung von Ziegeln ist eine derartige konkave Gestalt der Verbindungsflächen 12 üblich.

Fig. 3a zeigt ein Schnittbild durch einen Bausteinverbund 1 im Bereich zweier Bausteine 2 gemäß der vorliegenden Erfindung, wobei die Verbindungsflächen 12 der Bausteine 2 derart gestaltet oder ausgebildet sind, dass sich ein Klebespalt mit im Wesentlichen konstanter und möglichst geringer Breite d ergibt. Mit dem Begriff im Wesentlichen wird zum Ausdruck gebracht, dass selbstverständlich Oberflächenrauigkeiten der Verbindungsflächen 12 bis zu einem gewissen Ausmaß vorhanden sind, welche makroskopisch und mikroskopisch gesehen natürlich zu einer gewissen Schwankungsbreite der Breite d des Klebespalts führt. Ein Bausteinverbund 1 gemäß Figur 3a hat den Vorteil, dass wenig Verbindungsmaterial 3 für die Verbindung notwendig ist, und weiters durch die geringe Breite d des Klebespalts eine optimale Verbindung der Bausteine 2 mit ausgezeichneten Festigkeits- aber auch Dämmeigenschaften resultiert.

Fig. 3b zeigt eine Variante einer Verbindung zweier Bausteine 2, bei der die Verbindungsflächen 12 nicht eben sind, sondern zur Verhinderung eines seitlichen Verrutschens der Bausteine 2 mit komplementär gestalteten Ausformungen versehen sind. Wesentlich dabei ist, dass die Breite d des Klebespalts im Wesentlichen konstant ist und vorzugsweise einen bestimmten Maximalwert nicht überschreitet. Natürlich ist es im Rahmen der vorliegenden Erfindung möglich, dass entlang bestimmter Teilbereiche der Verbindungsflächen 12, wie z.B. den vertikal angeordneten Teilbereichen in Fig. 3b, die Breite d des Klebespalts von der Breite des restlichen Klebespalts abweicht.

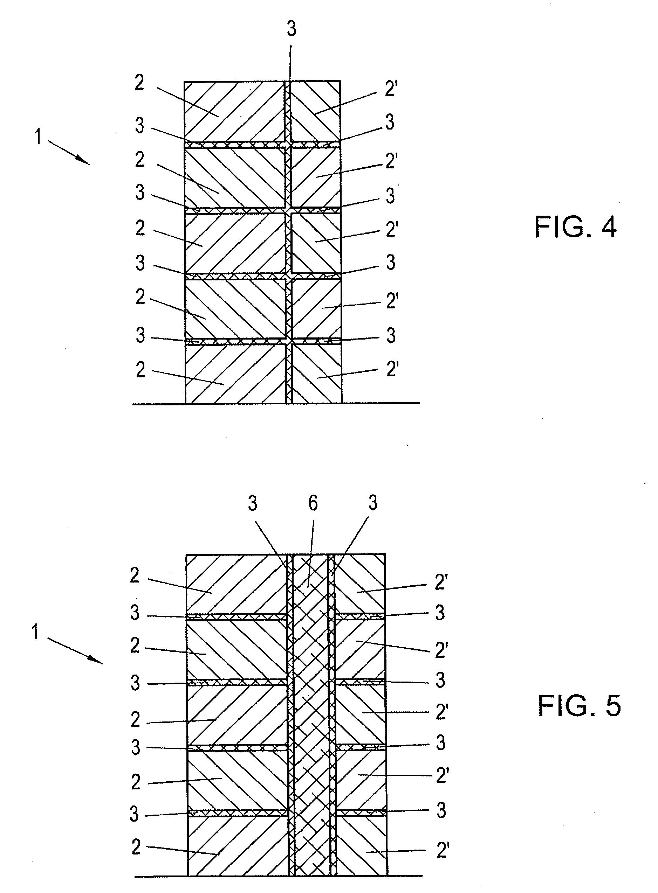

Fig. 4 zeigt einen Schnitt durch einen Bausteinverbund 1 bestehend aus mehreren übereinander und nebeneinander angeordneten Bausteinen 2, 2', so dass ein zweischaliger Bausteinverbund 1 hergestellt werden kann. Alle Bausteine 2, 2' sind mit dem erfindungsgemäßen Verbindungsmaterial 3, dem expandierbaren Kunststoffmaterial miteinander verbunden.

Fig. 5 zeigt einen gegenüber Fig. 4 erweiterten Bausteinverbund 1, bei dem zwischen zwei Bausteinverbunden eine Isolierschicht 6 angeordnet ist, welche ebenfalls mit dem Verbindungsmaterial 3 mit den Bausteinen 2, 2' verklebt ist. Durch Verwendung spezieller Isolierschichten 6, wie z.B. Vakuumisolationen, können auf diese Weise stabile Bausteinverbunde 1 mit hervorragenden Dämmeigenschaften erzielt werden. Beispielsweise konnte ein zweischaliger Bausteinverbund mit 25 cm breiten Hohlkammerziegeln, einem 4 cm dicken Vakuumisolationspaneel und einer weiteren Ziegelschicht aus 10 cm breiten Hohlkammerziegeln, welche mit Polyurethanschaum verbunden wurden, aufgebaut werden, der einen U-Wert von 0,1 W/m²K aufweist. Ein zweischaliger Bausteinverbund mit 30 cm breiten Hohlkammerziegeln, einem 4 cm dicken Vakuumisolationspaneel und einer weiteren Ziegelschicht aus 17 cm breiten Hohlkammerziegeln brachte sogar einen U-Wert von 0,09 W/m²K.

Ein derartiger U-Wert wäre mit herkömmlichen Bausteinverbunden nur bei Verwendung besonders dicker Isolationsschichten, wie z.B. 50 cm dicken Styroporschichten erzielbar.

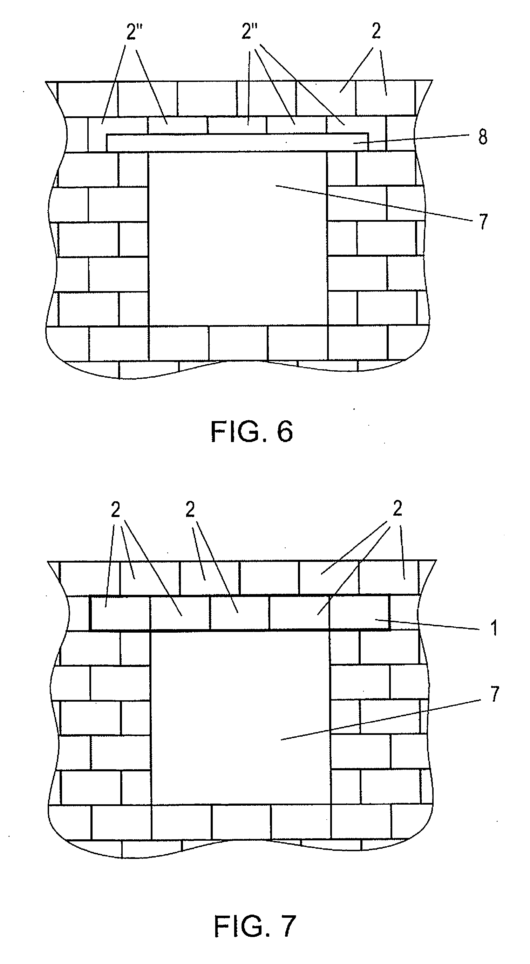

Fig. 6 zeigt eine Seitenansicht auf einen Teil einer aus einer Vielzahl von Bausteinen 2 aufgebauten Wand eines Gebäudes. In der Wand befindet sich eine Fensteröffnung 7, in welche ein Fenster eingebaut wird. Zur Bildung des oberen Abschlusses der Fensteröffnung 7 wird ein vorgefertigter sog. Sturz 8 auf die aufgemauerte Wand aufgelegt. Um dies zu ermöglichen, weist der Sturz 8 eine Länge auf, die größer ist als die Breite der Fensteröffnung 7. Da herkömmliche Stürze 8 meist Beton und allenfalls Metallverstärkungen enthalten, weisen sie relativ hohes Gewicht auf. Um dieses Gewicht in Grenzen zu halten, ist die Höhe des Sturzes 8 üblicherweise geringer als die Höhe der Bausteine 2. Dies erschwert naturgemäß die Herstellung einer mit einer Fensteröffnung 7 oder dergl. ausgestatteten Wand, da die direkt über dem Sturz 8 angeordneten Bausteine 2" entsprechend zugeschnitten werden müssen, so dass wieder eine Ebene für die darüberliegende Lage an Bausteinen 2 geschaffen wird.

Fig.7 zeigt schematisch die Anordnung einer Ausführungsform eines Bausteinverbunds 1 als obere Begrenzung einer Fensteröffnung 7 in einer aus einer Vielzahl von Bausteinen 2 aufgebauten Wand.

Der Bausteinverbund 1 ist vorzugsweise aus den selben Bausteinen 2 wie die übrige Wand hergestellt.

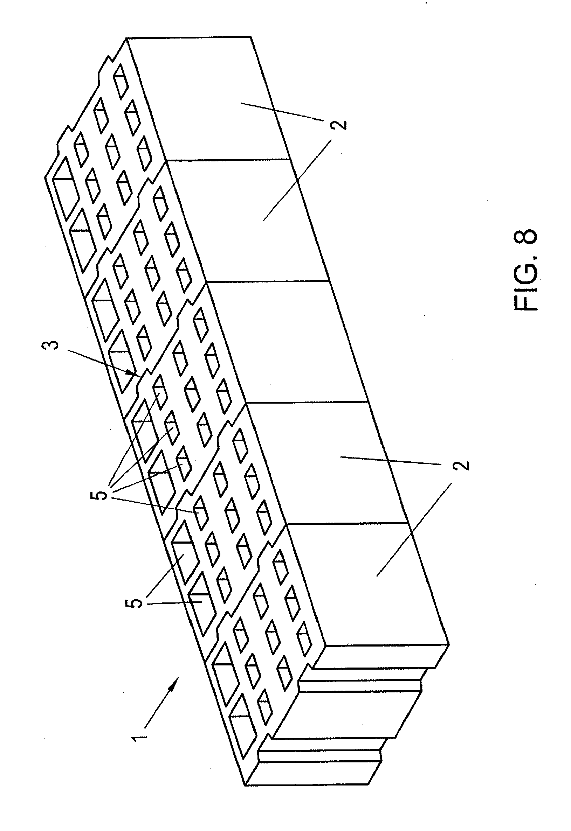

Fig. 8 zeigt eine perspektivische Ansicht eines derartigen Bausteinverbunds 1, der aus fünf Bausteinen 2, insbesondere Ziegeln, aufgebaut ist. Die Bausteine 2 weisen Hohlkammern 5 auf, welche einerseits das Gewicht der Bausteine 1 reduzieren und andererseits die Wärme- und Schalldämmung verbessern. In den Hohlkammern 5 können auch Dämmelemente angeordnet sein (nicht dargestellt). Erfindungsgemäß werden die Bausteine 1 mittels eines Verbindungsmaterials 3, das durch ein expandierbares Kunststoffmaterial gebildet ist, miteinander verbunden.

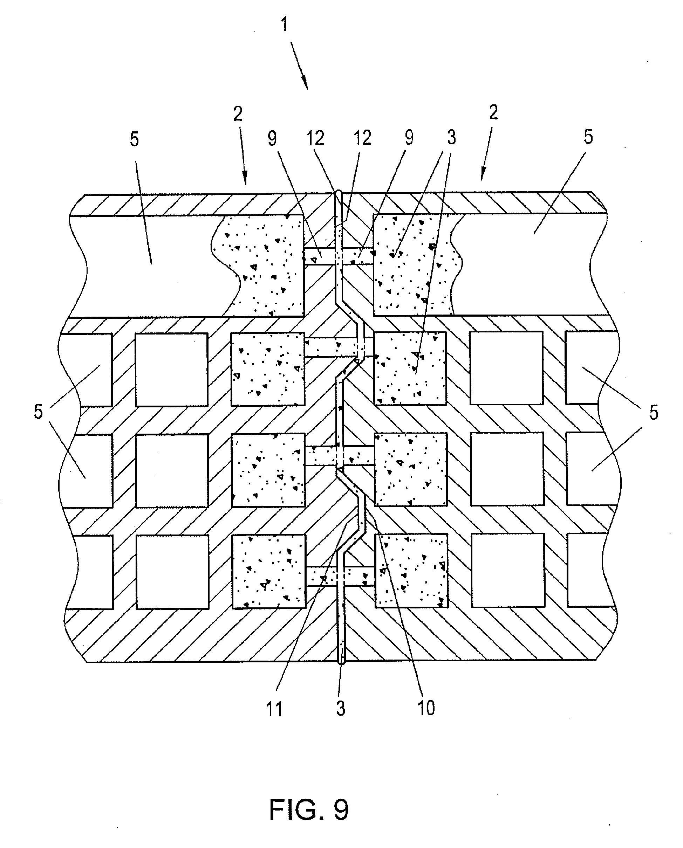

Das Detail gemäß Fig. 9 zeigt einen horizontalen Schnitt durch einen Teil des Bausteinverbunds 1 gemäß Fig. 8 im Bereich zweier nebeneinander angeordneter Bausteine 2. An den gegenüberliegenden Verbindungsflächen 12 der Bausteine 2 können Öffnungen 9 angeordnet sein, die vorzugsweise bis zu den hinter den Verbindungsflächen 12 angeordneten Hohlkammern 5 reichen. Das Verbindungsmaterial 3 ist sowohl an den Verbindungsflächen 12 der Bausteine 2 als auch in den Öffnungen 9 und teilweise in den Hohlkammern 5 angeordnet. Dadurch wird in Verbindung mit dem verwendeten expandierbaren Material eine besonders gute und stabile Verbindung zwischen den Bausteinen 2 erzielt. Zusätzlich dient das Verbindungsmaterial 7 in den Hohlkammern 5 als Dämmung. Auch die nicht mit den Öffnungen 9 in Verbindung stehenden Hohlkammern 5 können mit dem Verbindungsmaterial 3 ausgefüllt werden, um die Wärme- und Schalldämmung des Bausteinverbunds 1 zu erhöhen. Die Bausteine 2 können an den einander gegenüberliegenden Verbindungsflächen 12 komplementär gestaltete Verbindungselemente aufweisen, welche beispielsweise durch Nuten 10 und entsprechende Stege 11 gebildet sein können.

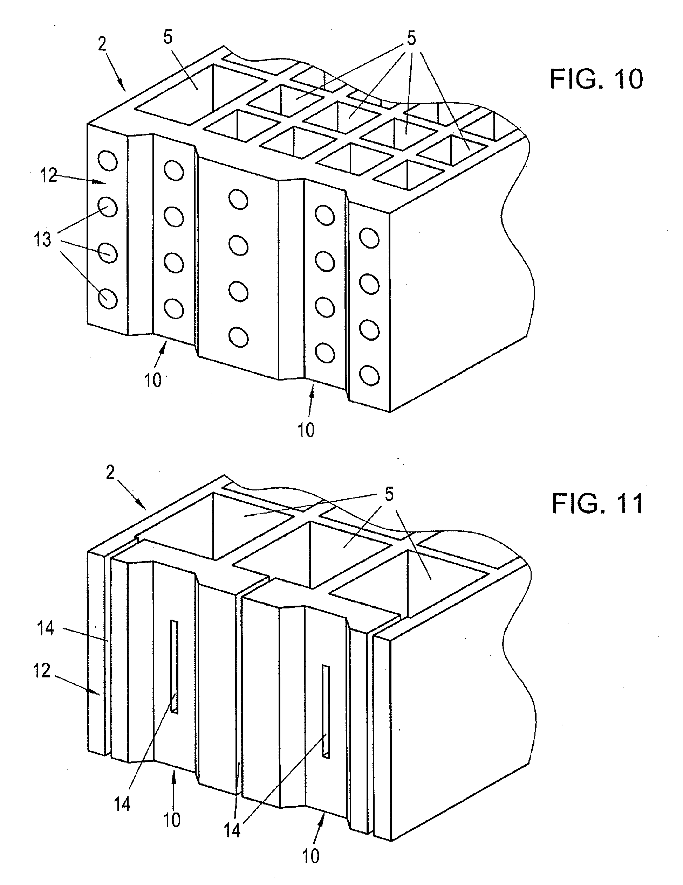

Fig.10 zeigt ein Ausführungsbeispiel eines Bausteins 2 in perspektivischer Ansicht auf die Verbindungsfläche 12. An der Verbindungsfläche 12 können Nuten 10 angeordnet sein, in welche entsprechende Stege 11 eines zu verbindenden Bausteins 2 eingreifen und somit eine bessere und sicherere Verbindung erzielt werden. Die Öffnungen 9 sind durch Bohrungen 13 gebildet, welche vorzugsweise bis zu den hinter der Verbindungsfläche 12 liegenden Hohlkammern 5 reichen, so dass das Verbindungsmaterial 3 über die Bohrungen 13 zumindest teilweise in die Hohlkammern 5 eindringen kann und eine Art Widerlager aufbauen kann.

Bei der Ausführungsvariante gemäß Fig. 11 sind die Öffnungen 9 durch Schlitze 14 gebildet, welche vorzugsweise ebenfalls bis zu den Hohlkammern 5 hinter der Verbindungsfläche 12 reichen, so dass das Verbindungsmaterial 3 über die Schlitze 14 bis zu den Hohlkammern 5 vordringen kann. Die Schlitze 14 können über die gesamte Höhe des Bausteins 2 reichen oder auch nur über einen Teil der Höhe des Bausteins 2 verlaufen.

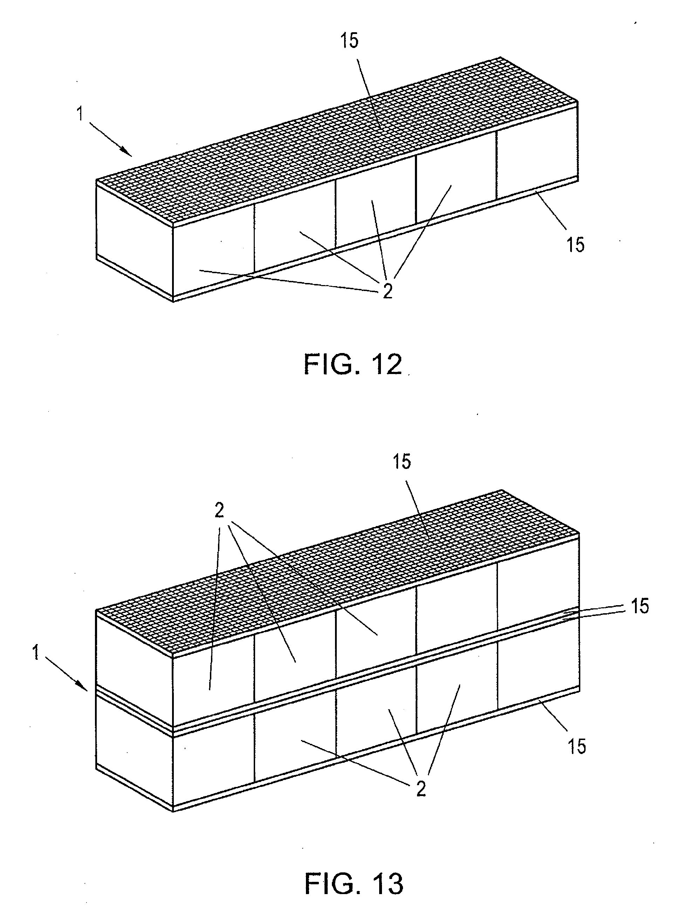

Fig.12 zeigt eine perspektivische Ansicht eines Bausteinverbunds 1, bestehend aus einigen Bausteinen 2, welche gemäß obiger Beschreibung miteinander verbunden sind. Zur Verstärkung des Bausteinverbunds 1 ist ein Verstärkungsgewebe 15, welches beispielsweise durch ein Glasfasergewebe gebildet ist, an der Ober- und bzw. oder Unterseite des Bausteinverbunds 1 angeordnet. Das Verstärkungsgewebe 15 wird vorzugsweise mit dem selben Verbindungsmaterial 3, das zur Verbindung der Bausteine 2 verwendet wird, aufgebracht.

Fig.13 zeigt eine schematische perspektivische Ansicht einer Anordnung zweier Bausteinverbunde 1 gemäß Fig.12, wodurch eine höhere Tragfähigkeit erzielt werden kann.

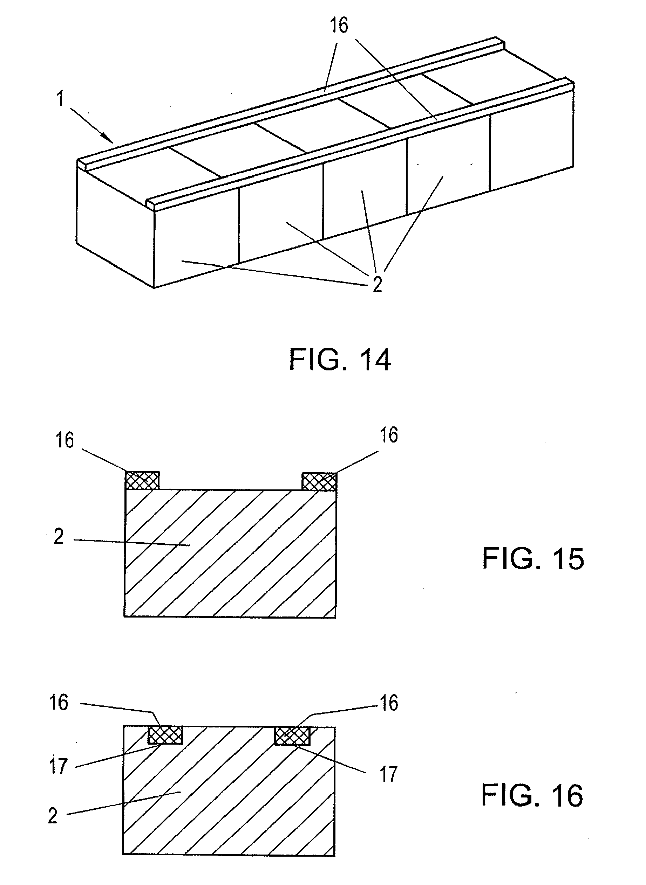

Fig. 14 zeigt eine perspektivische Ansicht eines Bausteinverbunds 1 bestehend aus einigen Bausteinen 2, wobei an der Oberseite des Bausteinverbunds 1 zwei Verstärkungsleisten 16 angeordnet sind.

Wie Fig. 15 zu entnehmen ist, können die Verstärkungsleisten 16 an der Oberseite des Bausteinverbunds 1 angeordnet und vorzugsweise angeklebt sein.

Bei der Variante gemäß Fig. 16 werden in der Oberfläche des Bausteinverbunds 1 Nuten 17 angeordnet, in welche die Verstärkungleisten 16 eingebracht werden. Dabei resultiert eine im

Wesentlichen ebene Oberfläche des Bausteinverbunds 1.

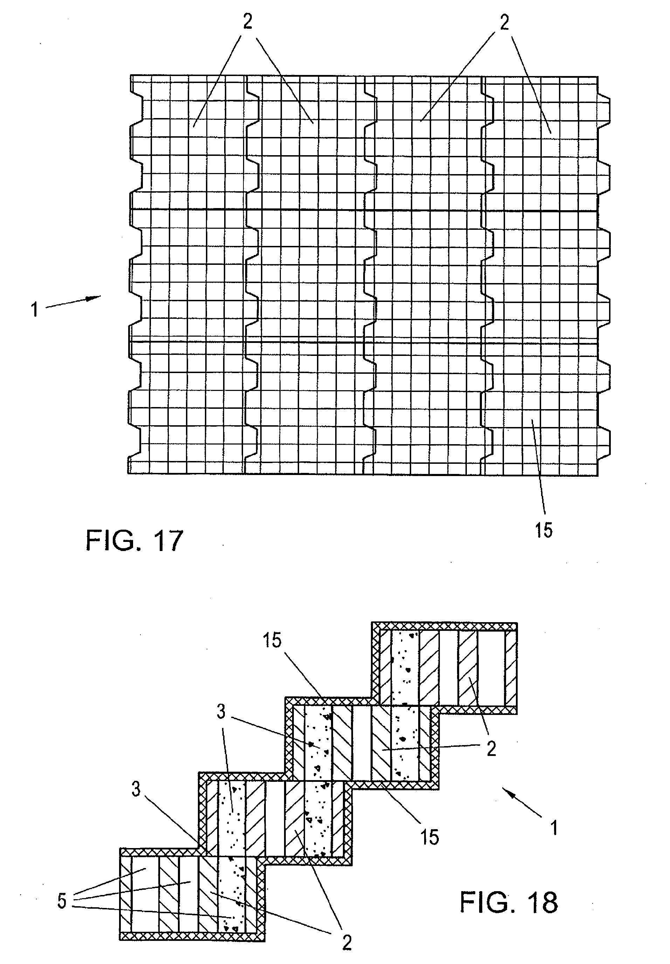

Fig. 17 zeigt die Draufsicht auf einen Bausteinverbund 1, bei dem mehrere Bausteine 2 in einer Ebene nebeneinander angeordnet werden, um eine Decke oder ein Deckenelement zu bilden. Über den nebeneinander angeordneten Bausteinen 2 kann wiederum ein Verstärkungsgewebe 15 angeordnet sein.

Schließlich zeigt Fig.18 eine Anordnung mehrerer Bausteine 2, welche teilweise überlappend miteinander verklebt werden, wodurch eine Treppe oder ein Treppenelement gebildet werden kann. Dabei wird an den Verbindungsflächen der Bausteine 2 das Verbindungsmaterial 3 aufgetragen, das auch in die Hohlkammern 5 reicht, und somit eine stabile Verbindung schafft. An der Ober- und bzw. oder Unterseite des Bausteinverbunds 1 kann wiederum ein Verstärkungsgewebe 15 aufgeklebt werden.

Es können gemäß der vorliegenden Erfindung verschiedene Bausteinverbunde lediglich unter Zu-Hilfe-Nahme üblicher Bausteine 2, insbesondere Ziegel, und unter Verwendung des expandierbaren Kunststoffmaterials als Verbindungsmaterial 3 hergestellt werden. Die Bausteinverbunde können aus jeglichen Bausteinformaten, insbesondere Ziegelformaten aufgebaut sein.

Im Folgenden wird anhand eines weiteren Beispiels noch die Festigkeit derartiger Bausteinverbunde erläutert. Es wurden drei Bausteinverbunde aus Planziegeln mit den Maßen 25-50 cm, zu je 2 m² aufgebaut. Die Planziegeln wurden in versetzter Anordnung aufeinander gesetzt, wobei als Verbindungsmaterial herkömmlicher Polyurethanschaum eingesetzt wurde. Dabei wurde der Schaum sowohl punktuell als auch entlang einer Linie, sowie auch flächig auf die Oberfläche der Planziegeln aufgespritzt. Es wurden drei Wände bei Temperaturen von +18°C, +8°C und -5°C aufgebaut. Zur Prüfung des Zusammenhalts des Bauwerkes wurde nach einer Aushärtezeit von etwa einer ½ h bis 1 h des PUR-Schaums die Wand mittels eines Krans aufgehoben. Es konnten keine Risse oder abgelöste Ziegel beobachtet werden; hingegen blieben die einzelnen Bausteine im Verbund. Die durch den Kran aufgebrachten Zugkräfte beliefen sich auf etwa 3 t/m². Als weiterer Test zur Festigkeit sowie Belastbarkeit wurde nach Aushärtung des PUR-Schaums mit einem Schlaghammer auf die Wand eingeschlagen. Diesen Kräften haben lediglich Teile der Ziegel nachgegeben, nicht jedoch der verbindende Polyurethanschaum.

Weitere Tests zeigten auch eine Eignung derart aufgebauter Bausteinverbunde in Erdbebengebieten. Darüber hinaus wurden gute Randeigenschaften erzielt, beispielsweise konnten mit erfindungsgemäß aufgebauten Bausteinverbunden die Brandschutzklassen F180 erzielt werden, wobei eine Erfüllung der Brandschutzklassen F60 bis F90 ausgereicht hätte. Lediglich an der Seite des Bausteinverbunds, die dem Feuer zugekehrt war, fand eine thermische Zersetzung des Verbindungsmaterials statt. In Bereichen an jener Seite des Bausteinverbunds, welche dem Feuer abgewandt war, blieb das Verbindungsmaterial unverändert erhalten.

## Patentansprüche

1. Verfahren zum Verbinden von Bausteinen mit Hohlkammern (5) zur Bildung eines Bausteinverbundes, wobei ein im Endzustand festes Verbindungsmaterial in fließfähigem Zustand auf zumindest eine Verbindungsfläche eines Bausteins aufgebracht wird und ein zu verbindender Baustein an diese Verbindungsfläche angelegt oder aufgesetzt wird, wobei als Verbindungsmaterial (3) ein expandierbares Kunststoffmaterial auf Basis von Polyurethan auf zumindest eine Verbindungsfläche (12) aufgebracht wird, **dadurch gekennzeichnet, dass** die zu verbindenden Bausteine (2) mit derartigen Verbindungsflächen (12) ausgestattet und aneinandergereiht werden, dass sich ein Klebespalt mit im Wesentlichen konstanter Breite (d) von kleiner 1 mm ergibt, und dass gleichzeitig mit dem Aufbringen des Verbindungsmaterials (3) auf zumindest eine Verbindungsfläche (8) des zu verbindenden Bausteins (2) auch die Hohlräume (5) in den Bausteinen (2) mit dem Verbindungsmaterial (3) befüllt werden.

2. Verfahren nach Anspruch 1, **dadurch gekennzeichnet, dass** zumindest eine Verbindungsfläche (12), vorzugsweise jede Verbindungsfläche (12) jedes zu verbindenden Bausteins (2) vor dem Aufbringen des Verbindungsmaterials (3) geschliffen wird.

3. Verfahren nach Anspruch 1 oder 2, **dadurch gekennzeichnet, dass** das Verbindungsmaterial (3) aus zumindest zwei Komponenten besteht, welche vor dem Aufbringen auf die Verbindungsfläche (12) in Kontakt gebracht werden.

4. Verfahren nach einem der Ansprüche 1 bis 3, **dadurch gekennzeichnet, dass** dem Verbindungsmaterial (3) vor dem Aufbringen auf die Verbindungsfläche (12) Zusätze beigemengt werden.

5. Verfahren nach einem der Ansprüche 1 bis 4, **dadurch gekennzeichnet, dass** das Verbindungsmaterial (3) punktuell auf zumindest eine Verbindungsfläche (12) des zu verbindenden Bausteins (2) aufgebracht wird.

6. Verfahren nach einem der Ansprüche 1 bis 4, **dadurch gekennzeichnet, dass** das Verbindungsmaterial (3) entlang zumindest einer Linie auf zumindest eine Verbindungsfläche (12) des zu verbindenden Bausteins (2) aufgebracht wird.

7. Verfahren nach einem der Ansprüche 1 bis 4, **dadurch gekennzeichnet, dass** das Verbindungsmaterial (3) flächig auf zumindest eine Verbindungsfläche (12) des zu verbindenden Bausteins (2) aufgebracht wird.

8. Verfahren nach einem der Ansprüche 1 bis 7, **dadurch gekennzeichnet, dass** zumindest eine Verbindungsfläche (8) des zu verbindenden Bausteines (2) vor dem Aufbringen des Verbindungsmaterials (3) vorbehandelt, beispielsweise angefeuchtet, wird.

9. Verfahren nach einem der Ansprüche 1 bis 8, **dadurch gekennzeichnet, dass** die Bausteine (2) mit zumindest einer Isolierschicht (6) verbunden werden, wobei als Verbindungsmaterial (3) vorzugsweise das expandierbare Kunststoffmaterial verwendet wird.

10. Verfahren nach einem der Ansprüche 1 bis 9, **dadurch gekennzeichnet, dass** die Bausteine (2) zu einem mehrschaligen Bausteinverbund (1) verbunden werden, wobei zwischen den einzelnen Bausteinverbunden (1) zumindest eine Isolierschicht (6) angeordnet wird, wobei die Isolierschicht (6) mit den Bausteinen (2) und allenfalls mehrere Isolierschichten (6) untereinander mit dem expandierbaren Kunststoffmaterial verbunden werden.

11. Verfahren nach einem der Ansprüche 1 bis 10, **dadurch gekennzeichnet, dass** der durch die Bausteine (2) gebildete Bausteinverbund (1) durch Aufbringen von Verstärkungselementen, wie zum Beispiel von Verstärkungsgeweben (15) oder Verstärkungsleisten (16) entlang zumindest einer Oberfläche des Bausteinverbunds (1) verstärkt wird.

12. Verfahren nach Anspruch 11, **dadurch gekennzeichnet, dass** in zumindest eine Oberfläche des Bausteinverbunds (1) zumindest eine Nut (17) od. dgl. zur Aufnahme eines Verstärkungselements, beispielsweise einer Verstärkungsleiste (16) eingebracht wird.

13. Verfahren nach Anspruch 11 oder 12, **dadurch gekennzeichnet, dass** die Verstärkungselemente (15, 16) mit dem Bausteinverbund (1) vorzugsweise unter Verwendung des expandierbaren Kunststoffmaterials verklebt werden.

14. Bausteinverbund (1) mit einer Vielzahl von einzelnen, mittels eines Verbindungsmaterials (3) in Form eines expandierten Kunststoffmaterials auf Basis von Polyurethan-Schaum miteinander verbundenen Bausteinen (2) mit Hohlkammern (5), **dadurch gekennzeichnet, dass** der Klebespalt zwischen zwei verbundenen Bausteinen (2) eine im Wesentlichen konstante Breite (d) von maximal 1 mm aufweist, und dass das Verbindungsmaterial (3) auch die Hohlkammern (5) der Bausteine (2) befüllt.

15. Bausteinverbund nach Anspruch 14, **dadurch gekennzeichnet, dass** zumindest eine Verbindungsfläche (12) der verbundenen Bausteine (2) geschliffen ist.

16. Bausteinverbund nach Anspruch 14 oder 15, **dadurch gekennzeichnet, dass** die Bausteine (2) mit einem Mehrkomponenten-Material verbunden sind.

17. Bausteinverbund nach einem der Ansprüche 14 bis 16, **dadurch gekennzeichnet, dass** die Bausteine (2) an den Verbindungsflächen (8) Öffnungen (9) zur Aufnahme des Verbindungsmaterials (3) aufweisen.

18. Bausteinverbund nach Anspruch 17, **dadurch gekennzeichnet, dass** die Öffnungen (9) an den Verbindungsflächen (12) zumindest teilweise bis zu Hohlkammern (5) reichen, und dass das Verbindungsmittel (3) zumindest teilweise in den Hohlkammern (5) angeordnet ist.

19. Bausteinverbund nach einem der Ansprüche 14 bis 18, **dadurch gekennzeichnet, dass** zumindest die mit den Öffnungen (9) verbundenen Hohlkammern (5) vollständig mit dem Verbindungsmaterial (3) ausgefüllt sind.

20. Bausteinverbund nach einem der Ansprüche 14 bis 19, **dadurch gekennzeichnet, dass** zumindest an einer Oberfläche der verbundenen Bausteine (2) zumindest ein Verstärkungselement, beispielsweise ein Verstärkungsgewebe (15) oder eine Verstärkungsleiste (16) angeordnet und mit diesem verklebt ist.

21. Bausteinverbund nach Anspruch 20, **dadurch gekennzeichnet, dass** an der Oberfläche, an der das zumindest eine Verstärkungselement angeordnet ist, eine Nut (17) od. dgl. zur Aufnahme des Verstärkungselements, insbesondere der Verstärkungsleiste (16) angeordnet ist.

22. Bausteinverbund nach Anspruch 20 oder 21, **dadurch gekennzeichnet, dass** an der Ober- und Unterseite der miteinander verbundenen Bausteine (2) jeweils zumindest ein Verstärkungselement angeordnet ist.

23. Bausteinverbund nach einem der Ansprüche 14 bis 22, **dadurch gekennzeichnet, dass** mit den Bausteinen (2) zumindest eine Isolierschicht (6) mit dem expandierbaren Kunststoffmaterial verbunden ist.

24. Bausteinverbund nach einem der Ansprüche 14 bis 23, **dadurch gekennzeichnet, dass** zwischen Bausteinen (2) zumindest eine Isolierschicht (6) angeordnet ist, wobei die Isolierschicht (6) mit den Bausteinen (2) und allenfalls mehrere Isolierschichten (6) untereinander mit dem expandierbaren Kunststoffmaterial verbunden sind.

## Claims

1. A method for connecting building blocks with voids (5) for the formation of a building-block assembly, wherein a joining material solid in its final state is applied in its free-flowing state to at least one joining surface of a building block, and a building block to be connected is joined or fitted to said joining surface, with a polyurethane-based expandable plastics material being applied on at least one joining surface (12) as joining material (3), **characterized in that** the building blocks (2) to be connected are provided with such joining surfaces (12) and joined together, that an adhesive gap having a substantially constant width (d) smaller than 1 mm is formed, and that simultaneously with the application of the joining material (3) on at least one joining surface (8) of the building block (2) to be connected also the voids (5) in the building blocks (2) are filled with the joining material (3).

2. A method according to claim 1, **characterized in that** at least one joining surface (12), preferably each joining surface (12), of every building block (2) to be connected is ground prior to applying the joining material (3).

3. A method according to claim 1 or 2, **characterized in that** the joining material (3) is comprised of at least two components which are contacted prior to being applied to the joining surface (12).

4. A method according to any one of claims 1 to 3, **characterized in that** the joining material (3) is supplemented with additives prior to being applied on the joining surface (12).

5. A method according to any one of claims 1 to 4, **characterized in that** the joining material (3) is spotted on at least one joining surface (12) of the building block (2) to be connected.

6. A method according to any one of claims 1 to 4 **characterized in that** the joining material (3) is applied along at least one line on at least one joining surface (12) of the building block (2) to be connected.

7. A method according to any one of claims 1 to 4, **characterized in that** the joining material (3) is planarly applied on at least one joining surface (12) of the building block (2) to be connected.

8. A method according to any one of claims 1 to 7, **characterized in that** at least one joining surface (8) of the building block (2) to be connected is pretreated, e.g. moistened, prior to applying the joining material (3).

9. A method according to any one of claims 1 to 8, **characterized in that** the building blocks (2) are connected with at least one insulating layer (6), with the expandable plastics material being preferably used as joining material (3).

10. A method according to any one of claims 1 to 9, **characterized in that** the building blocks (2) are joined to form a multi-shell building block assembly (1), wherein at least one insulating layer (6) is arranged between the individual building block assemblies (1), said insulating layer (6) with the building blocks (2), and optionally several insulating layers (6) with one another, being connected by the expandable plastics material.

11. A method according to any one of claims 1 to 10, **characterized in that** the building block assembly (1) formed by the building blocks (2) is reinforced by applying reinforcing elements such as, e.g., reinforcing tissues (15) or reinforcing ledges (16), along at least one surface of the building block assembly (1).

12. A method according to claim 11, **characterized in that** at least one groove (17) or the like is introduced into at least one surface of the building block assembly (1) for receiving a reinforcing element, e.g. a reinforcing ledge (16).

13. A method according to claim 11 or 12, **characterized in that** the reinforcing elements (15, 16) are glued to the building block assembly (1), preferably by using the expandable plastics material.

14. A building block assembly (1) including a plurality of individual building blocks (2) with voids (5), which are joined together by a joining material (3) in the form of a polyurethane-foam-based expandable plastics material, **characterized in that** the adhesive gap between two joined building blocks (2) has a substantially constant width (d) of 1 mm at most, and that the joining material (3) also fills the voids (5) of the building blocks (2).

15. A building block assembly according to claim 14, **characterized in that** at least one joining surface (12) of the joined building blocks (2) is ground.

16. A building block assembly according to claim 14 or 15, **characterized in that** the building blocks (2) are joined by a multi-component material.

17. A building block assembly according to any one of claims 14 to 16, **characterized in that** the building blocks (2) comprise openings (9) for receiving the joining material (3) on their joining surfaces (8).

18. A building block assembly according to claim 17, **characterized in that** the openings (9) on the joining surfaces (12) reach at least partially as far as to the voids (5), and that the joining material (3) is at least partially arranged within the voids (5).

19. A building block assembly according to any one of claims 14 to 18, **characterized in that** at least the voids (5) connected with the openings (9) are completely filled with the joining material (3).

20. A building block assembly according to any one of claims 14 to 19, **characterized in that** at least one reinforcing element, e.g. a reinforcing tissue (15) or a reinforcing ledge (16) is arranged on, and glued to, at least one surface of the building blocks (2) to be connected.

21. A building block assembly according to claim 20, **characterized in that** a groove (17) or the like for receiving the reinforcing element, in particular the reinforcing ledge (16), is provided on the surface on which the at least one reinforcement element is arranged.

22. A building block assembly according to claim 20 or 21, **characterized in that** at least one reinforcing element is each arranged on the respective upper and lower sides of the connected building blocks (2).

23. A building block assembly according to any one of claims 14 to 22, **characterized in that** at least one insulating layer (6) is connected with the building blocks (2) by the expandable plastics material.

24. A building block assembly according to any one of claims 14 or 23, **characterized in that** at least one insulating layer (6) is arranged between building blocks (2), said insulating layer (6) with the building blocks (2), and optionally several insulating layers (6) with one another, being connected by the expandable plastics material.

## Revendications

1. Procédé pour assembler des parpaings avec des chambres creuses (5) pour réaliser une construction de parpaings, un matériau d'assemblage solide à l'état final étant appliqué à l'état coulant sur au moins une surface d'assemblage d'un parpaing et un parpaing à assembler étant posé ou placé sur cette surface d'assemblage, comme matériau d'assemblage (3) étant appliqué un matériau synthétique expansible à base de polyuréthane sur au moins une surface d'assemblage (12), **caractérisé en ce que** les parpaings (2) à assembler sont équipés de telles surfaces d'assemblage et sont juxtaposés qu'il résulte une fente de collage avec une largeur (d) essentiellement constante de moins d' 1 mm, et qu'en même temps que l'application du matériau d'assemblage (3) sur au moins une surface d'assemblage (8) du parpaing à assembler (2), les espaces creux (5) dans les parpaings (2) soient aussi remplis de matériau d'assemblage (3).

2. Procédé selon la revendication 1, **caractérisé en ce qu'**au moins une surface d'assemblage (12), de préférence chaque surface d'assemblage (12) de chaque parpaing (2) à assembler est polie avant l'application du matériau d'assemblage (3).

3. Procédé selon la revendication 1 ou 2, **caractérisé en ce que** le matériau d'assemblage (3) se compose d'au moins deux composants qui sont amenés en contact avant l'application sur la surface d'assemblage (12).

4. Procédé selon l'une quelconque des revendications 1 à 3, **caractérisé en ce que** des additifs sont ajoutés au matériau d'assemblage (3) avant l'application sur la surface d'assemblage (12).

5. Procédé selon l'une quelconque des revendications 1 à 4, **caractérisé en ce que** le matériau d'assemblage (3) est appliqué ponctuellement sur au moins une surface d'assemblage (12) du parpaing (2) à assembler.

6. Procédé selon l'une quelconque des revendications 1 à 4, **caractérisé en ce que** le matériau d'assemblage (3) est appliqué le long d'au moins une ligne sur au moins une surface d'assemblage (12) du parpaing (2) à assembler.

7. Procédé selon l'une quelconque des revendications 1 à 4, **caractérisé en ce que** le matériau d'assemblage (3) est appliqué à plat sur au moins une surface d'assemblage (12) du parpaing (2) à assembler.

8. Procédé selon l'une quelconque des revendications 1 à 7, **caractérisé en ce qu'**au moins une surface d'assemblage (8) du parpaing (2) à assembler est prétraitée avant l'application du matériau d'assemblage (3), par exemple humidifiée.

9. Procédé selon l'une quelconque des revendications 1 à 8, **caractérisé en ce que** les parpaings (2) sont reliés à au moins une couche d'isolation (6), comme matériau d'assemblage (3) étant utilisé de préférence le matériau synthétique expansible.

10. Procédé selon l'une quelconque des revendications 1 à 9, **caractérisé en ce que** les parpaings (2) sont assemblés pour former une construction de parpaings (1) à multicoques, entres les constructions de parpaing individuelles (1) étant disposée au moins une couche d'isolation (6), la couche d'isolation (6) étant reliée aux parpaings (2) et éventuellement plusieurs couches d'isolation (6) étant reliées entre elles avec le matériau synthétique expansible.

11. Procédé selon l'une quelconque des revendications 1 à 10, **caractérisé en ce que** la construction de parpaings (1) formée par les parpaings (2) est renforcée par l'application d'éléments de renfort tels que des tissus de renforcement (15) ou barres de renfort (16) le long d'au moins une surface de la construction de parpaings (1).

12. Procédé selon la revendication 11, **caractérisé en ce que** dans au moins une surface de la construction de parpaings (1) est introduite au moins une rainure (17) ou similaire pour la réception d'un élément de renfort, par exemple d'une barre de renfort (16).

13. Procédé selon la revendication 11 ou 12, **caractérisé en ce que** les éléments de renfort (15, 16) sont collés à la construction de parpaings (1) de préférence en utilisant le matériau synthétique expansible.

14. Construction de parpaings (1) avec une pluralité de parpaings (2) individuels assemblés entre eux à l'aide d'un matériau d'assemblage (3) sous la forme d'un matériau synthétique à base de mousse de polyuréthane avec des chambres creuses (5), **caractérisée en ce que** la fente de collage entre deux parpaings (2) assemblés présente une largeur (d) essentiellement constante d' 1 mm maximum et **en ce que** le matériau d'assemblage (3) remplit aussi les chambres creuses (5) des parpaings (2).

15. Construction de parpaings selon la revendication 14, **caractérisée en ce qu'**au moins une surface d'assemblage (12) des parpaings assemblés (2) est polie.

16. Construction de parpaings selon 1a revendication 14 ou 15, **caractérisée en ce que** les parpaings (2) sont assemblés avec un matériau à plusieurs composants.

17. Construction de parpaings selon l'une quelconque des revendications 14 à 16, **caractérisée en ce que** les parpaings (2) sur les surfaces d'assemblage (8) présentent des ouvertures (9) pour la réception du matériau d'assemblage (3).

18. Construction de parpaings selon 1a revendication 17, **caractérisée en ce que** les ouvertures (9) sur les surfaces d'assemblage (12) atteignent au moins en partie les chambres creuses (5) et **en ce que** le moyen d'assemblage (3) est disposé au moins en partie dans les chambres creuses (5).

19. Construction de parpaings selon l'une quelconque des revendications 14 à 18, **caractérisée en ce qu'**au moins les chambres creuses (5) assemblées aux ouvertures (9) sont complètement remplies du matériau d'assemblage (3).

20. Construction de parpaings selon l'une quelconque des revendications 14 à 19, **caractérisée en ce qu'**au moins sur une surface des parpaings assemblés (2), au moins un élément de renfort, par exemple un tissu de renforcement (15) ou une barre de renfort (16) est disposée et collée à celle-ci.

21. Construction de parpaings selon la revendication 20, **caractérisée en ce que** sur la surface, sur laquelle est disposé l'au moins un élément de renfort, est disposée une rainure (17) ou similaire pour la réception de l'élément de renfort, en particulier de la barre de renfort (16).

22. Construction de parpaings selon la revendication 20 ou 21, **caractérisée en ce que** le côté supérieur et inférieur des parpaings (2) assemblés entre eux est disposé respectivement au moins un élément de renfort.

23. Construction de parpaings selon l'une quelconque des revendications 14 à 22, **caractérisée en ce qu'**aux parpaings (2) est assemblée au moins une couche d'isolation (6) avec le matériau synthétique expansible.

24. Construction de parpaings selon l'une quelconque des revendications 14 à 23, **caractérisée en ce qu'**entre les parpaings (2) est disposée au moins une couche d'isolation (6), la couche d'isolation (6) étant assemblée aux parpaings (2) et éventuellement plusieurs couches d'isolation (6) étant assemblées entre elles avec le matériau synthétique expansible.
